(19) Europäisches Patentamt
European Patent Office
Office européen des brevets

(11) EP 0 978 236 A1

# (12) EUROPÄISCHE PATENTANMELDUNG

(43) Veröffentlichungstag:
**09.02.2000 Patentblatt 2000/06**

(51) Int. Cl.[7]: **A23K 1/16**, A61K 31/05

(21) Anmeldenummer: **99115698.5**

(22) Anmeldetag: **09.08.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **07.08.1998 DE 19835850**

(71) Anmelder:
**BASF AKTIENGESELLSCHAFT**
**67056 Ludwigshafen (DE)**

(72) Erfinder:
- **Krämer, Klaus, Dr.**
  **76829 Landau (DE)**
- **Baldenius, Kai-Uwe, Dr.**
  **67227 Frankenthal (DE)**
- **Hoppe, Peter Paul, Dr.med.vet.**
  **67157 Wachenheim (DE)**

(74) Vertreter:
**Kinzebach, Werner, Dr. et al**
**Patentanwälte**
**Reitstötter, Kinzebach und Partner**
**Postfach 86 06 49**
**81633 München (DE)**

## (54) Verwendung von Hemmstoffen der Cholesterin-Biosynthese zur Senkung des Cholesteringehaltes in Geflügelei

(57) Die Erfindung betrifft die Verwendung wenigstens eines Cholesterin-Biosynthese-Hemmers zur Verringerung des Cholesteringehaltes in Geflügelei, wobei der Hemmer ein isoprenoides Strukturelement umfasst.

EP 0 978 236 A1

## Beschreibung

Die Erfindung betrifft die Verwendung von Hemmstoffen der Cholesterin-Biosynthese bzw. der Hydroxymethylglutaryl (HMG)-Coenzym A (CoA)-Reduktase zur Verringerung des Cholesteringehaltes in Geflügelei sowie Futtermittelzusammensetzungen für Geflügel, welche solche Hemmstoffe enthalten.

Bei Mensch und Tier ist Cholesterin wesentlicher Bestandteil von Zellmembranen und dient als Ausgangssubstanz für die Synthese von Steroidhormonen und Gallensäuren. Endogenes und exogenes Cholesterin (Nahrungscholesterin) können gleichermaßen für die intermediären Stoffwechselvorgänge herangezogen werden.

Hypercholesterinämie (>200 mg/dl) ist neben Rauchen, Übergewicht, Hypertonie und Hyperhomocysteinämie Risikofaktor koronarer Herzerkrankungen. Der Beitrag von alimentärem Cholesterin zur Atherogenese ist noch immer Gegenstand intensiver Diskussion. Die Beeinflussung des Plasmacholesterins durch Nahrungscholesterin ist sehr variabel. Stoffwechselgesunde reagieren kaum auf die Erhöhung der Cholesterinzufuhr. Ein Teil der Bevölkerung (in den USA 36 %) reagiert auf exogenes Cholesterin mit einem ausgeprägten Plasmacholesterinanstieg. Bei dieser Bevölkerungsgruppe scheint neben einer höheren Absorptionsrate, ein gestörter Feedback-Mechanismus der endogenen Cholesterinsynthese vorzuliegen. Nach epidemiologischen Untersuchungen vermindert ein um 1 % niedrigerer Plasmacholesterinspiegel das Risiko koronarer Herzerkrankungen um 2 % (Palca, Science 247, 1170-1171, 1990).

Maßnahmen zur Cholesterinsenkung sind eine Reduzierung der Fettzufuhr, insbesondere von gesättigten tierischen Fetten. Die damit einhergehende Cholesterinbegrenzung steht erst an zweiter Stelle. Weiterhin sollte der Anteil an einfach oder mehrfach ungesättigten Fettsäuren in der Nahrung erhöht werden, wobei der lipidsenkenden Wirkung der ω-3-Fettsäuren eine besondere Bedeutung zukommt (Deutsche Gesellschaft für Ernährung, Beratungsstandards, 1995).

Nach neueren Erkenntnissen scheint Cholesterin per se nicht der alleinige Risikofaktor zu sein, sondern vielmehr auch oxidierte Lipide, einschließlich Oxicholesterine in der LDL-Fraktion (Morel, D. W. und Lin Y. C., Nutr. Biochem. 7, 495-506, 1996). Die Erhöhung der Aufnahme antioxidativer Substanzen, insbesondere von Vitamin E, ist daher nach mehreren Autoren ein wünschenswerter präventiver Ansatz zur Vermeidung ischämischer Erkrankungen (Holvoet, P. und Collen D., FASEB J. 8, 1279-1284, 1994, Jha, P. et al., Ann. Int. Med., 123, 11, 860-872, 1995).

Trotz der mittlerweile differenzierten Betrachtungsweise von Nahrungacholesterin bestehen von nationalen Expertenkommitees, z. B. Deutsche Gesellschaft für Ernährung, Nutrition Research Council der USA, Empfehlungen, auch für die Allgemeinbevölkerung, die tägliche Cholesterinzufuhr auf 300 mg zu begrenzen.

Insgesamt hat die Cholesterindiskussion zu einer hochgradigen Verunsicherung der Bürger hinsichtlich des Nähr- und Gesundheitswerts von Eiern geführt. Der Verbrauch an Eiern in Deutschland ist daher innerhalb von 10 Jahren von 280 auf 218 Stück pro Kopf 1994/95 zurückgegangen.

Aus Untersuchungen beim Menschen ist bekannt, dass Hafer- und Reiskleie sowie Gerste eine lipidsenkende Wirkung aufweisen (Poulter N., et al., Am. J. Clin. Nutr., 58-66-9, 1993; Dubois C. et al., Am. J. Clin. Nutr. 61, 325-333, 1995; McIntosh G. H. et al., World Rev. Nutr. Diet 77, 89-108, 1995). Diesen Effekt hat man lange nur dem Gehalt an löslichen Nicht-Stärke-Polysacchariden (β-Glucane) zugeschrieben, die die Fettabsorption durch Adsorption von Gallensäuren und Erhöhung der Viskosität im Chymus reduzieren. Die cholesterinsenkende Wirkung dieser Getreidearten ist wahrscheinlich auch auf ihren Gehalt an Tocotrienolen (hauptsächlich α-Tocotrienol) zurückzuführen (Qureshi, A. A.. et al., J. Biol. Chem. 261, 23, 10544-10550, 1986; Hegstedt, M. und Kousik, C. S., Louisiana Agriculture 36, 2, 16-17, 1994; Qureshi, N. und Qureshi, A. A., in: Packer, L. und Fuchs, J. (Hrsg.), Vitamin E in Health and Disease. Marcel Dekker, New York, 1993, 247-267; Lia et al., Am. J. Clin. Nutr. Diet 62, 1245-1251, 1995). Tocotrienole hemmen das Schlüsselenzym der Cholesterinbiosynthese, die β-Hydroxy-β-methyl-glutaryl (HMG)-CoA-Reduktase in der Leber (posttranskriptionale Down-Regulation). Als wirksamstes Isomer wurde in HepG2-Zellkulturen γ-Tocotrienol (ca. 30fach stärker als α-Tocotrienol) identifiziert. Keine Unterschiede bestanden zwischen der racemischen (synthetisschen) und natürlichen (chiralen) Form. Im Vergleich zur Alkohol- und Acetatform war die Wirkung des Nicotinats am ausgeprägtesten (US 5,217,992).

Versuche, den Cholesteringehalt von Geflügeleiern durch diätetische Maßnahmen zu beeinflussen, waren bislang wenig erfolgreich (Beyer, R. S. und Jensen, L. S., Poultry Sci. 72, 1339-1348, 1989; Naber, E. C. , Feedstuffs 62, 5, 48-52, 1990; Gerken, M., Jahrbuch für Geflügelwirtschaft, Verlag Eugen Ulmer, Stuttgart, 1994, 138-143; Grashorn, M. A., Feed Mix 3, 4, 28-31, 1995). Mit synthetischen HMG-CoA-Reduktase-Hemmern, sog. Statinen, gelang jedoch eine Senkung um 15 % (Lovastatin) bzw. 30 % ((±)-(R*,R*)-3,4-Dibromo-β,δ-dihyroxy-2-(4-fluorophenyl)-5-(1-methylethyl)-1H-pyrrol-1-heptansäure, Na-Salz), ohne die Leistungsparameter des Geflügels, wie die Legeleistung, zu beeinflussen (Elkin, R. G. und Rogler, J. C., J. Agric. Food Chem. 38, 1635-1641, 1990; Elkin, R. G. et al., J. Agric. Food Chem. 41, 1094-1101, 1993). Statine sind jedoch synthetische Arzneimittel, so dass bei deren Verfütterung die Gefahr einer unerwünschten Lokalisierung von Syntheserückständen im Ei besteht. In einer anderen Untersuchung blieb Simvastatin allerdings ohne Wirkung auf den Cholesteringehalt im Ei (Huggett, C. D. et al., Biochem. Soc. Trans. 21, 147, 1993).

Die Cholesterinsenkung durch Tocotrienole bei Mensch, Broiler und hypercholesterinämischem Schwein

wurde in der US 5,217,992 untersucht. Versuche zur Cholesterinsenkung in Geflügelei wurden nicht beschrieben. Nach einer Untersuchung von Beyer, R. S. und Jensen, L. (Poultry Sci. 68, 1703-1706, 1993) hat α-Tocotrienol jedenfalls keine Wirkung auf den Cholesteringehalt im Ei. Die Legehenne ist auf eine Mindestsynthese an Cholesterin angewiesen, da Cholesterin für die Synthese von Sexualhormonen benötigt wird. Der Östrogenspiegel ist dementsprechend bei Hennen besonders hoch. Auch das Küken benötigt einen Mindestcholesteringehalt des Eidotters während der Embryonalentwicklung. Es kann daher nicht ausgeschlossen werden, dass eine erzwungene Cholesterinsenkung negative Effekte in Bezug auf Legeleistung der Legehennen und/oder Embryonalentwicklung der Küken zeigt.

Für eine Reihe von Abkömmlingen (pflanzliche Terpenoide) des Mevalonatweges (Cholesterinbiosynthese), z. B. für Linalool, Geraniol und d-Limonen, aber auch für β-Ionon, wurde bei Chemopräventionsexperimenten in Zellkultur bzw. an Labortieren, wie Ratte und Maus, HMG-CoA-Reduktase-Hemmung und damit einhergehend eine Tumorinhibierung nachgewiesen (Elson, C., Am. Inst. Nutr. 1666-1672, 1995; Yu, S. G. et al., Am. Chem. Soc. 42, 7, 1493-1498, 1994; Yu, S. G. et al., Am. Inst. Nutr. 2763-2767, 1995; Elson, C. E. und Yu, S. G., Am. Inst. Nutr., 607-614, 1994). Auch die Carotinoide Lycopin und β-Carotin bewirken im Makrophagen eine Hemmung der Cholesterinsynthese (Fuhrmann et al., Biochem. Biophys. Res. Commun. 233, 658-662, 1997). Eine mögliche Brauchbarkeit dieser Verbindungen für die Senkung des Cholesteringehalts in Geflügelei wird in dieser Publikation jedoch nicht vorgeschlagen und wurde bisher auch nicht untersucht.

Es ist deshalb Aufgabe der vorliegenden Erfindung, einen neuen Weg zur Verringerung des Cholesteringehaltes in Geflügelei zu finden.

Überraschenderweise konnte obige Aufgabe gelöst werden durch Verwendung wenigstens eines Hemmers als Futtermittelzusatz für Geflügel, wobei der Hemmer ein isoprenoides Strukturelement umfasst. Die erfindungsgemäß wirksamen Substanzen sind im weitesten Sinne Cholesterin-Biosynthese-Hemmer. Da ihre Wirkung über eine Hemmung der HMG-CoA Reduktase erklärbar ist (ohne daran gebunden zu sein) werden sie im Rahmen der Erfindung auch als HMG-CoA-Reduktase-Hemmer bezeichnet.

Im Rahmen der vorliegenden Erfindung versteht man unter Substanzen mit isoprenoidem Strukturelement Verbindungen, die ein Kohlenstoffgrundgerüst mit isoprenoidem Bauprinzip aufweisen, also aus Isopreneinheiten, d. h. $C_5$-Bausteinen aufgebaut oder davon abgeleitet sind. Außerdem versteht man darunter solche Verbindungen, die wenigstens eine Teilstruktur umfassen, welche ein solches isoprenoides Bauprinzip besitzt. Dieses isoprenoide Strukturelement ist außerdem gekennzeichnet durch das Vorhandensein wenigstens einer Kohlenstoff-Kohlenstoff-Doppelbindung. Beispiele für isoprenoide Strukturelemente sind solche, die von Terpen (d. h. $C_{10}H_{16}$), Sesquiterpen (d. h. $C_{15}H_{24}$) oder Tetraterpen ($C_{40}H_{64}$) abgeleitet sind.

Beispiele für erfindungsgemäß brauchbare HMG-CoA-Reduktase-Hemmer sind Tocotrienole sowie Terpenoide. Bevorzugte Terpenoide sind pflanzliche Terpenoide, insbesondere solche mit Terpen-, Sesquiterpen- oder Tetraterpenstruktur oder einer davon abgeleiteten Struktur. Insbesondere sind zu nennen Hemmer mit Terpen- oder Sesquiterpenstruktur, und vor allem Verbindungen ausgewählt unter Iononen, Linaloolen, Farnesylen und Perillylalkohol.

Als Beispiele für Tocotrienole sind zu nennen: α-, β-, γ- und δ-Tocotrienol, deren 4-Oxo-Analoga sowie die Ester dieser Verbindungen.

Tocotrienole besitzen folgende allgemeine Formel:

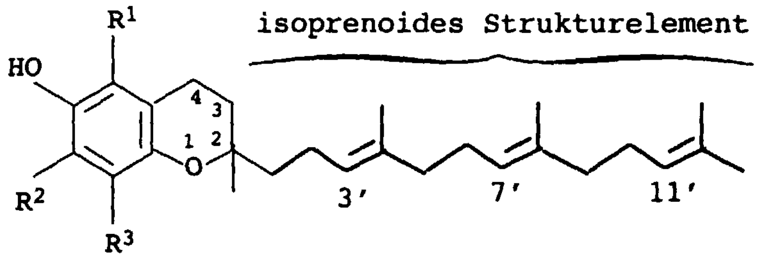

α-Tocotrienol: $R^1 = R^2 = R^3 = CH_3$
β-Tocotrienol: $R^1 = R^3 = CH_3$, $R^2 = H$
γ-Tocotrienol: $R^1 = H$, $R^2 = R^3 = CH_3$
δ-Tocotrienol: $R^1 = R^2 = H$, $R^3 = CH_3$

4-Oxo-Analoga davon weisen in 4-Position eine Ketogruppe auf. Als Beispiel hierfür kann genannt werden: 4-Oxo-γ-tocotrienol. Geeignete Ester obiger Tocotrienole sind über die 6-OH-Gruppe beispielsweise mit einer ein- oder

zweiwertigen, gesättigten oder ein- oder mehrfach ungesättigten $C_2$-$C_{22}$-Carbonsäure verestert. Beispiele für geeignete kurzkettige Säuren sind $C_2$-$C_6$-Carbonsäuren, wie z. B. Ameisensäure, Essigsäure, Propionsäure oder Bernsteinsäure. Beispiele für längerkettige Säuren sind Citronensäure, Palmitinsäure, Stearinsäure, Ölsäure, Linolsäure, α- und γ-Linolensäure, Dihomo-γ-linolensäure, Arachidonsäure, Eicosapentaensäure und Docosahexaensäure. Weiterhin geeignet sind gesättigte oder ungesättigte heteroatomhaltige Carbonsäuren, wie z. B. α-Liponsäure oder Nicotinsäure.

Als Beispiele für erfindungsgemäß brauchbare Terpenoide sind insbesondere pflanzliche Terpenoide zu nennen. Insbesondere sind die pflanzlichen Terpenoide ausgewählt unter Terpenen (Verbindungen abgeleitet von einer $C_{10}H_{16}$-Struktur), wie z. B. Linalool-, Ionon- und Perillyl-Verbindungen; oder Sesquiterpenen (d. h. Verbindungen abgeleitet von einer $C_{15}H_{24}$-Struktur), wie z. B. Farnesol-Verbindungen.

Ionone besitzen folgende allgemeine Formel:

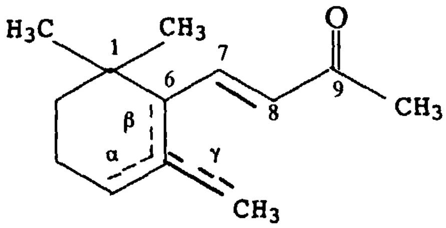
,

wobei je nach Lage der Doppelbindung zwischen α-, β- und γ-Ionon zu unterscheiden ist.

Erfindungsgemäß brauchbare Linaloole sind darstellbar durch folgende Formel:

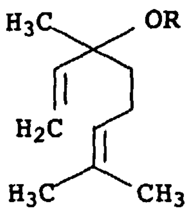

R = H : Linalool
R = CO-Acyl: Linalylester,

wobei als Beispiel für einen Linalylester Linalyl-$C_2$-$C_6$-Carbonsäureester, wie z. B. Linalylacetat, zu nennen ist.

Der erfindungsgemäß ebenfalls brauchbare Perillylalkohol besitzt folgende Strukturformel:

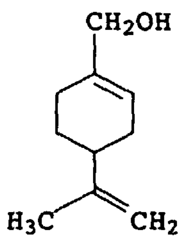

Als erfindungsgemäß brauchbare Farnesyle sind zu nennen: Farnesol der Formel

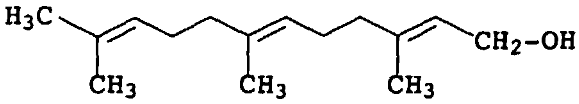

sowie die davon abgeleiteten Ester. Als geeignete Ester sind zu nennen Ester von ein- oder zweiwertigen, gesättigten oder ein- oder mehrfach ungesättigten $C_2$-$C_{22}$-Carbonsäuren. Beispiele für geeignete kurzkettige Säuren sind $C_2$-$C_6$-Carbonsäuren, wie Ameisensäure, Essigsäure, Propionsäure oder Bernsteinsäure. Beispiele für längerkettige Säuren sind Citronensäure, Palmitinsäure, Stearinsäure, Olsäure, Linolsäure, α- und γ-Linolensäure, Dihomo-γ-linolensäure, Arachidonsäure, Eicosapentaensäure und Docosahexaensäure. Weiterhin geeignet sind gesättigte oder ungesättigte beteroatomhaltige Carbonsäuren, wie z. B. α-Liponsäure oder Nicotinsäure.

Beispiele für erfindungsgemäß brauchbare Tetraterpene sind Carotinoide. Aus der Gruppe der Carotinoide sind insbesondere zu nennen Carotine, wie z. B. α-, β-, γ-, δ-, ε- oder ψ,ψ-Carotin (Lycopin) oder all-trans-Lycopin, vor allem aber β-Carotin und Lycopin.

Die erfindungsgemäßen Hemmstoffe des oben bezeichneten Typs werden im Gemisch mit herkömmlichen Futtermitteln dem jeweiligen Geflügel verabreicht. Die Konzentration des Hemstoffes variiert natürlich in Abhängigkeit vom jeweils verwendeten Wirkstoff, der Art des Geflügels und der Fütterungsweise, liegt aber etwa im Bereich von 5 bis 10 000 mg/kg Futter. Die jeweils optimale Wirkstoffkonzentration kann jedoch vom Durchschnittsfachmann ohne weiteres bestimmt werden. Typische Gehaltsbereiche (bezogen auf das Gesamtgewicht des Futters in kg) für einzelne der oben genannten Hemmer sind:

| | |
|---|---|
| Tocotrienole: | 5 - 5000 mg/kg Futter |
| Oxo-Tocotrienole: | 5 - 5000 mg/kg Futter |
| Linaloole: | 10 - 5000 mg/kg Futter |
| β-Ionon: | 10 - 5000 mg/kg Futter |
| Farnesol: | 20 - 7500 mg/kg Futter |
| Perillylalkohol: | 5 - 7500 mg/kg Futter |
| Carotinoide: | 5 - 7500 mg/kg Futter |

Die vorliegende Erfindung umfasst die Verwendung oben genannter Hemmstoffe in isomerenreiner Form, in optisch reiner Form, als Isomerengemisch oder Stereoisomerengemisch oder aber auch als Gemisch von Isomeren und Stereoisomeren.

Gemäß einer bevorzugten Ausführungsform, werden solche Hemmstoffe verwendet, welche sowohl zu einer Cholesterinabnahme in Geflügelei als auch zu einer Triglyceridabnahme im Geflügelei und/oder Plasma führen. Als Beispiel hierfür sind zu nennen γ-Tocotrienolester, wie z. B.γ-Tocotrienolacetat.

Mit erfindungsgemäß brauchbaren Hemmern lassen sich gewöhnlich Verringerungen im Cholesteringehalt des Eidotters von bis zu etwa 40 %, vorzugsweise von etwa 10 bis etwa 30 % erreichen. Erfindungsgemäß zu beobachtende Verringerungen des Triglyceridgehalts im Eidotter bzw. im Plasma liegen im Bereich von etwa 30 %, vorzugsweise etwa 10 bis 20 %. Diese Werte erreicht man gewöhnlich nach einer Fütterungsdauer von etwa 10 Tagen.

Gegenstand der Erfindung sind außerdem Futtermittelzusammensetzungen für Geflügel, welche in Kombination mit üblichen Futtermittelbestandteilen wenigstens einen der oben bezeichneten HMG-CoA-Reduktase-Hemmer enthalten. Als typische Futtermittelbestandteile sind zu nennen: Mais, Gerste, Weizen, Hafer, Roggen, Tritikale, Sorghum, Reis und Kleien, Grießkleien sowie Mehle dieser Getreidearten, Sojabohnen, Sojaprodukte wie Sojaextraktionsschrot, Raps, Rapsextraktionsschrot, Baumwollsaat und Extraktionsschrot, Sonnenblumen, Sonnenblumenextraktionsschrot, Leinsaat, Leinextraktionsschrot, Expeller von Ölsaaten, Ackerbohnen, Erbsen, Gluten, Gelatine, Tapioka, Hefen, Single Cell Protein, Fleischknochenmehl, Fleischmehl, Blutmehl, Fischmehl, Salze, Mineralstoffe, Spurenelemente, Vitamine, Aminosäuren, Futterfett, Öle (Soja, Raps, Sonnenblume etc.).

Die vorliegende Erfindung wird nun unter Bezugnahme auf die beiliegenden Figuren näher beschrieben. Dabei zeigt

Figur 1 ein Balkendiagramm der ermittelten Cholesteringehalte in Hühnereidotter bei Fütterung verschiedener HMG-CoA-Reduktase-Hemmer sowie Vitamin E und Nikotinsäure. Die Zuordnung der einzelnen Balken ist wie folgt: 1: Kontrolle; 2: Vitamin E, 1000 mg/kg; 3: γ-Tocotrienolacetat, 20 mg/kg; 4: γ-Tocotrienolacetat, 500 mg/kg; 5: d-Limonen, 5000 mg/kg; 6: Nikotinsäure, 1500 mg/kg; und 7: Nikotinsäure, 15 000 mg/kg (Angabe jeweils in mg Hemmer je kg Futter). Ein über einem Balken angegebener Pfeil deutet eine Verminderung (↓) bzw. eine Erhöhung (↑) des Cholesteringehaltes bei p<0,05 an;

Figur 2 ein Balkendiagramm der ermittelten Triglyceridgehalte im Ei bei Fütterung von HMG-CoA-Reduktase-Hemmern, Vitamin E und Nikotinsäure. Die Zuordnung der Balken entspricht der Figur 1.

Beispiel 1

a) Futterformulierung

Es wurde ein mehlförmiges Versuchsfutter auf Basis von Mais und Tapioka mit der in Tabelle 1 angegebenen Zusammensetzung hergestellt. Gerste, Hafer, Weizen und Roggen wurden nicht verwendet, weil diese relevante Mengen an Tocotrienolen enthalten. Als Fettquelle und Träger essentieller Fettsäuren diente Sojaöl, das wie Maiskeimöl und Olivenöl kein Tocotrienol enthält (vgl. Qureshi & Qureshi, 1993, a.a.O.).

Tabelle 1

| Zusammensetzung und analysierte Nährstoffgehalte der Versuchsdiät | |
|---|---|
| Zusammensetzung | % |
| Mais | 48.000 |
| Tapioka | 16.000 |
| HP-Sojaschrot | 18.000 |
| Stärke | 2.000 |
| Weizengrießkleie | 1.430 |
| Salz | 0.350 |
| Futterkalk | 7.900 |
| Cefkaphos (MCP)[1] | 1.300 |
| Vitamin-VM[2] | 0.400 |
| Spurenelement-VM | 0.020 |
| Cholinchlorid 70 % | 0.120 |
| Sojaöl | 4.000 |
| Luprosil Salz | 0.400 |
| Nährstoffgehalt | % |
| Wasser | 9.90 |
| Rohprotein | 14.10 |
| Rohfett | 7.30 |
| Rohfaser | 3.00 |
| Rohasche | 12.30 |
| Gesamtzucker | 3.00 |
| Rohstärke | 41.70 |
| Natrium | 0.15 |
| Calcium | 3.79 |
| Phosphor | 0.61 |
| ME (MJ/kg)[3] | 12.00 |

1) Cefkaphos (MCP) = Monocalciumphosphat
2) VM = Vormischung
3) ME = umsetzbare (metabolisierbare) Energie

b) Versuchsplan

Die untersuchten Wirkstoffe wurden in 2 Dosierungen einer Futtermittelformulierung gemäß Tabelle 1 zugesetzt, mit Ausnahme von Vitamin E (all-rac-α-Tocopherylacetat), das nur in der Dosierung von 1000 mg/kg verwendet wurde (vgl. Tabelle 2).

Tabelle 2

| Versuchsplan 1 | | |
|---|---|---|
| Behandlung Nr. | Wirkstoff | Dosierung im Futter [mg/kg] |
| 1 | - | - |
| 2 | (all-rac-α-Tocopherylacetat) | 1000[a] |
| 3 | γ-Tocotrienolacetat, synth. | 20[a] |
| 4 | γ-Tocotrienolacetat, synth. | 500 |
| 5 | d-Limonen | 5000[b] |
| 6 | d-Limonen | 50000[b] |
| 7 | Nicotinsäure | 1500[c] |
| 8 | Nicotinsäure | 15000 |

[a] Wirksame Dosierung gemäß US 5,217,992
[b] Basiert auf chemopräventiver Wirkung bei 0,5 % Zusatz zur Diät (Elson und Yu, 1994, a.a.O.)
[c] Dosierungen von 150-300 X RDA (Recommended Dietary Allowance) sind beim Menschen wirksam (Bässler et al., 1992, a.a.O.)

Zusätzlich zu den Hemmstoffen der HMG-CoA-Reduktase wurden Nikotinsäure und Vitamin E (all-rac-α-Tocopherylacetat) verwendet. Die cholesterinsenkende Wirkung hoher Nikotinsäuredosen ist aus der Literatur seit langem bekannt (Bässler, K. H. et al., Vitamin-Lexikon für Ärzte, Apotheker und Ernährungswissenschaftler, Gustav Fischer Verlag, 1992). Beim Broiler wurde durch Vitamin E-Supplementierung eine Stimulierung der HMG-CoA-Reduktase und bei gleichzeitiger Cholesterinfütterung ein Anstieg des Plasmacholesterins beobachtet (Qureshi, A. A. et al., Nutr. Rep. Int. 40, 5, 992-1001, 1989). Dieser Effekt sollte im vorliegenden Versuch an der Legehenne überprüft werden.

Zu Versuchsbeginn befanden sich die Legehennen (Lohmann Selected Leghorn) in der 28. Legewoche. Eine Behandlungsgruppe bestand aus 10 Hennen. Die Tiere wurden über 4 wochen mit den verschiedenen Wirkstoffen angefüttert. Danach wurden die Eier für die Analysen gesammelt. Es wurden 3 unabhängige Eidotterpools von je 6 Eiern gebildet und bis zur Analyse tiefgefroren. Gesamtcholesterin und Triglyceride im Eidotter wurden nach enzymatischen Methoden analysiert. Die Analysen wurden entsprechend den Angaben in dem Handbuch "Veterinärmedizinische Laboruntersuchungen für die Diagnose und Verlaufskontrolle" (1985), 3. Aufl, Hrsg. Boehringer Mannheim GmbH, Mannheim durchgeführt.

Für die Cholesterinbestimmung werden die Cholesterinester unter Einwirkung der Cholesterin-Esterase in Cholesterin und Fettsäure gespalten. Das freie Cholesterin wird von Luftsauerstoff unter Mitwirkung von Cholesterin-Oxidase zu $\Delta^4$-Cholestenon und Wasserstoffperoxid umgesetzt. Das entstehende Wasserstoffperoxid bildet mit 4-Aminophenazon und Phenol unter katalytischer Wirkung der Peroxidase einen roten Farbstoff, dessen Farbintensität der Cholesterin-Konzentration direkt proportional ist und bei 500 bis 550 nm gemessen werden kann.

Die Triglyceride werden enzymatisch über ihren Glyceringehalt bestimmt. Die Spaltung der Triglyceride erfolgt enzymatisch durch die Enzyme Lipase und Esterase. Das entstandene Glycerin wird in der von Glycerinkinase (GK) katalysierten Reaktion durch ATP zu Glycerin-3-phosphat phosphoryliert.

$$\text{Glycerin} + \text{ATP} \leftrightarrows \text{Glycerin-3-phosphat} + \text{ADP}$$

Mittels Pyruvatkinase (PK) wird das entstandene ADP durch Phosphoenolpyruvat (PEP) unter Bildung von Pyruvat wieder in ATP überführt.

$$\text{PEP} + \text{ADP} \overset{\text{PK}}{\leftrightarrows} \text{Pyruvat} + \text{ATP}$$

Pyruvat wird durch NADH mittels Lactatdehydrogenase (LDH) zu Lactat hydriert, wobei NADH zu NAD oxidiert wird.

$$\text{Pyruvat} + \text{NADH} + \text{H}^+ \overset{\text{LDH}}{\leftrightarrows} \text{Lactat} + \text{NAD}^+$$

Die während der Reaktion verbrauchte NADH-Menge ist äquivalent der Glycerin-Menge. NADH ist Messgröße und auf Grund seiner Absorption bei Hg 366 nm, Hg 334 nm oder 340 nm leicht zu bestimmen.

Tocopherole und Tocotrienole in den Eidotterpools wurden mittels HPLC bestimmt. Dazu wird die Probe in wässrig-alkoholischem Medium verseift und mehrmals mit Diethylether extrahiert. Die Quantifizierung erfolgt durch Vergleich mit externen Standards.

| | |
|---|---|
| Stationäre Phase: | Spherisorb CN 5 µm, 250 x 4 mm |
| Mobile Phase: | n-Hexan mit 0,25 % i-Propanol |
| Flussrate: | 2 ml/min |
| Fluoreszenzdetektion: | 292 nm (Extinktion) |
| | 326 nm (Emission) |
| UV/VIS: | 326 nm. |

Typische Retentionszeiten betragen für:

| | |
|---|---|
| α-Tocopherol | 3,74 min |
| β-Tocopherol | 12,15 min |
| γ-Tocopherol | 12,93 min |
| β-Tocotrienol | 14,22 min |
| γ-Tocotrienol | 16,49 min |

Am Versuchsende wurde ein Geruchstest mit den rohen und gekochten Eiern durchgeführt.

Die Daten wurden varianzanalytisch ausgewertet und Mittelwertunterschiede nach dem Duncan-Test rangiert.

c) Ergebnisse

Der Geruchstest ließ keine Abweichungen von der Kontrolle erkennen.

Die ermittelten Cholesterin- und Triglyceridgehalte sind in den Figuren 1 und 2 dargestellt. Da zwischen den Dottergewichten keine signifikanten Unterschiede bestanden, sind die Gehalte bezogen auf den Gesamtdotter angegeben. Durch γ-Tocotrienolacetat, 500 mg/kg, wurden Cholesterin und Triglyceride signifikant um 15 % im Eidotter reduziert. Die niedrige Tocotrienoldosierung und Vitamin E (all-rac-α-Tocopherylacetat) hatten keinen Effekt auf diese Parameter. Der Triglyceridgehalt wurde durch beide Nikotinsäuredosierungen signifikant erhöht.

Die folgende Tabelle 3 zeigt die Tocopherol- und Tocotrienolgehalte im Eidotter von Behandlung 1, 2, 3 und 4.

Tabelle 3

| Tocopherol- und Tocotrienol-Gehalte im Eidotter bei Fütterung von Tocopherylacetat und Tocotrienolacetat | | | | | | | |
|---|---|---|---|---|---|---|---|
| Behandlung | Wirkstoff | Dosierung im Futter | α-Tocopherol | γ-Tocopherol | δ-Tocopherol | α-Tocotrienol | γ-Tocotrienol |
| | | [mg/kg] | [jeweils μg/Dotter] | | | | |
| 1 | Kontrolle | 0 | 481 | 399 | 66 | 0 | 0 |
| 2 | all-rac-α-Tocopherylacetat | 1000 | 23064 | 188 | 36 | 227 | 0 |
| 3 | R,S-γ-Tocotrienolacetat | 20 | 545 | 393 | 57 | 0 | 0 |
| 4 | R,S-γ-Tocotrienolacetat | 500 | 433 | 425 | 59 | 0 | 366 |

Es handelt sich dabei um die nach Verseifung ermittelten Werte. Der α-Tocopherolgehalt im Eidotter stieg von 0,48 mg in der Kontrollgruppe durch Vitamin E (all-rac-α-Tocopherylacetat, 1000 mg/kg) auf 23 mg an. Dies entspricht einer Transferrate von etwa 21 %, was gut mit Literaturwerten übereinstimmt (Sünder, A. et al., Proc. Soc. Nutr. Physiol. 6, 147, 1997). Darüber hinaus verursachte die hohe α-Tocopheroldosierung eine Verminderung des γ-Tocopherols im Dotter. Auch dieser Befund ist literaturbekannt. γ-Tocotrienol wurde überraschenderweise kaum im Eidotter angereichert. Lediglich bei der hohen Dosierung konnten 366 μg γ-Tocotrienol im Eidotter nachgewiesen werden. Die Transferrate in Eidotter betrug demnach nur ca. 0,008 %.

Beispiel 2

Analog zu Beispiel 1 wurde ein weiteres Fütterungsexperiment durchgeführt. Dabei sollten neben α- und δ-Tocotrienol, eine Tocotrienolvorstufe (4-Oxo-γ-tocotrienol), weitere Terpenoide, wie Farnesylacetat, Perillylalkohol, Linalool und β-Ionon, an der Legehenne geprüft werden.

a) Futterformulierung:

Mais-Tapioka-Soja (vgl. Beispiel 1) + 150 ppm Ethoxyquin (Antioxidans)
Linolsäuregehalt auf 1 % mit Sojaöl einstellen
Vitamin E-Zusatz nach NRC (Nutrition Research Council/USA) (1994): 5 ppm all-rac-α-Tocopherylacetat

b) Versuchsplan:

Der Versuchsplan entspricht dem aus Beispiel 1, jedoch mit folgenden Änderungen:

Die Tierzahl betrug 8 Hennen pro Behandlung.

Jeweils 4 Tiere aus der Kontrolle und den hohen Dosierungen werden zu Versuchsende getötet. Nach der Blutentnahme für die Plasmagewinnung wurden die Tiere vollständig entblutet. Die Leber wurde danach entnommen und bis zur Analyse tiefgefroren.

Triglycerid und Cholesterin wurde in 4 unabhängigen Eidotterpools (à 6 Eier) und Plasma bestimmt.

Außerdem wurden α-, δ- und 4-Oxo-γ-tocotrienol in Eidotter und Leber (Behandlungen 1 bis 7) bestimmt.

Der Versuchsplan ist in der folgenden Tabelle 4 dargestellt:

Tabelle 4

| Versuchsplan 2 | | |
|---|---|---|
| Behandlung | Substanz | Zusatz [mg/kg] |
| 1 | - | - |
| 2 | α-Tocotrienol | 50 |
| 3 | α-Tocotrienol | 500 |
| 4 | δ-Tocotrienol | 50 |
| 5 | δ-Tocotrienol | 500 |
| 6 | 4-Oxo-γ-tocotrienol | 50 |
| 7 | 4-Oxo-γ-tocotrienol | 500 |
| 8 | Linalool | 100 |
| 9 | Linalool | 1000 |
| 10 | β-Ionon | 100 |
| 11 | β-Ionon | 1000 |
| 12 | Farnesylacetat | 750 |
| 13 | Farnesylacetat | 7500 |
| 14 | Perillylalkohol | 500 |
| 15 | Perillylalkohol | 5000 |

c) Ergebnisse

Der Versuch verlief planungsgemäß und störungsfrei. Es wurden infolge der Behandlungen weder Einbrüche bei der Legeleistung noch geruchliche Abweichungen der Eier festgestellt.

Die Versuchsergebnisse für die ermittelten Cholesterin- und Tri-glyceridgehalte sind in folgender Tabelle 5 zusammengefasst:

Tabelle 5

| Wirkstoff | Dosierung im Futter mg/kg | Cholesterin | | Triglyceride | |
|---|---|---|---|---|---|
| | | Eidotter [mg/g] | Plasma [mg/dl] | Eidotter [mg/g] | Plasma (mg/dl] |
| - | - | 10,23 | 125,3 | 170,4 | 2496 |
| α-Tocotrienol | 50 | 9,85 | | 189,9 | |
| α-Tocotrienol | 500 | 9,45 | 130,3 | 195,1 | 4195 |
| δ-Tocotrienol | 50 | 9,25 | | 185,2 | |
| δ-Tocotrienol | 500 | 9,23 | 163,0 | 163,9 | 2985 |
| 4-Oxo-γ-tocotrienol | 50 | 9,78 | | 188,3 | |
| 4-Oxo-γ-tocotrienol | 500 | 8,55 | 166,8 | 172,0 | 4336 |
| Linalool | 100 | 8,40 | | 187,5 | |
| Linalool | 1000 | 8,97 | 211,6 | 163,1 | 1907 |
| β-Ionon | 100 | 9,03 | | 190,7 | |
| β-Ionon | 1000 | 9,03 | 201,3 | 184,8 | 1954 |

Tabelle 5 (fortgesetzt)

| Wirkstoff | Dosierung im Futter mg/kg | Cholesterin | | Triglyceride | |
|---|---|---|---|---|---|
| | | Eidotter [mg/g] | Plasma [mg/dl] | Eidotter [mg/g] | Plasma (mg/dl] |
| Farnesylacetat | 750 | 10,15 | | 177,1 | |
| Farnesylacetat | 7500 | 10,23 | 129,3 | 175,9 | 1929 |
| Perillylalkohol | 500 | 9,55 | | 188,5 | |
| Perillylalkohol | 5000 | 9,53 | 165,3 | 174,4 | 2197 |

In den folgenden Tabellen 6 und 7 sind die in Eidotter bzw. Leber ermittelten Tocopherol- bzw. Tocotrienolgehalte zusammengefasst:

Tabelle 6

| Tocopherol- und Tocotrienolgehalte im Eidotter bei Fütterung von α- und δ-Tocotrienol sowie 4-Oxo-γ-Tocotrienol | | | | | | | | | |
|---|---|---|---|---|---|---|---|---|---|
| Wirkstoff | Dosierung in Futter [mg/kg] | α-Tocopherol | β-Tocopherol | γ-Tocopherol | δ-Tocopherol | α-Tocotrienol | γ-Tocotrienol | δ-Tocotrienol | 4-Oxo-γ-Tocotrienol |
| | | [jeweils μg/g] | | | | | | | |
| - | - | 19,36 | 0,94 | 14,54 | 1,89 | 0,21 | 0,00 | 0,00 | 5,75 |
| α-Tocotrienol | 50 | 32,32 | 1,97 | 17,34 | 1,83 | 16,86 | 0,00 | 0,00 | 4,88 |
| α-Tocotrienol | 500 | 93,86 | 8,22 | 16,31 | 1,67 | 150,28 | 0,19 | 0,00 | 0,00 |
| δ-Tocotrienol | 50 | 25,89 | 0,93 | 16,97 | 1,56 | 0,75 | 0,26 | 0,63 | 2,01 |
| δ-Tocotrienol | 500 | 29,29 | 0,91 | 17,24 | 1,63 | 3,49 | 0,70 | 3,77 | 2,02 |
| 4-Oxo-γ-tocotrienol | 50 | 25,35 | 0,36 | 16,83 | 1,25 | 0,75 | 0,00 | 0,00 | 3,67 |
| 4-Oxo-γ-tocotrienol | 500 | 24,43 | 0,00 | 19,18 | 1,62 | 1,28 | 0,00 | 0,00 | 10,73 |

Tabelle 7

| Tocopherol- und Tocotrienolgehalte in der Leber von Legehennen bei Fütterung von α- und δ-Tocotrienol sowie 4-Oxoy-γ-Tocotrienol | | | | | | | | | |
|---|---|---|---|---|---|---|---|---|---|
| Wirkstoff | Dosierung in Futter [mg/kg] | α-Tocopherol | β-Tocopherol | γ-Tocopherol | δ-Tocopherol | α-Tocotrienol | γ-Tocotrienol | δ-Tocotrienol | 4-Oxo-γ-Tocotrienol |
| | | [jeweils µg/g] | | | | | | | |
| - | - | 2,73 | n. n. | 1,46 | n. n. | 0,66 | n. n. | n. n. | n. n. |
| α-Tocotrienol | 500 | 11,82 | 1,25 | 2,64 | n. n. | 11,06 | n. n. | n. n. | n. n. |
| δ-Tocotrienol | 500 | 4,37 | n. n. | 2,26 | n. n. | 0,92 | n. n. | n. n. | n. n. |
| 4-Oxo-γ-tocotrienol | 500 | 4,15 | n. n. | 3,10 | n. n. | 1,00 | n. n. | n. n. | n. n. |

Obige Versuchsergebnisse zeigen, dass durch α- und δ-Tocotrienol sowie Perillylalkohol ein überraschender Trend zu verminderten Cholesteringehalten im Eidotter bestand. Überraschenderweise wurde auch durch 4-Oxo-γ-tocotrienol in der hohen Dosierung (500 mg/kg) und beiden Dosierungen der Terpenoide Linalool und β-Ionon das Eidotter-Cholesterin signifikant um bis zu 18 % vermindert. Die Behandlungen hatten keinen Effekt auf den Plasma-Cholesterin-Gehalt. Dagegen wurde der Plasma-Triglycerid-Spiegel durch β-Ionon, Farnesylacetat und Perillylalkohol um maximal 25 % reduziert. Die Triglyceride im Eidotter stiegen durch die meisten Behandlungen geringfügig an.

Durch die Tocotrienolbehandlungen stieg α-Tocopherol im Eidotter an. Die Ursache dafür ist, dass die verwendeten Präparate noch geringere Mengen an α-Tocopherol enthielten. β-Tocopherol wurde lediglich durch die Behandlungen mit α-Tocotrienol beeinflusst. Von den Tocotrienolen im Eidotter stieg am deutlichsten die α-Form an. Hochgerechnet auf Gesamtdotter betrug die Retention bei 500 mg/kg α-Tocotrienol 2,4 mg, wodurch sich eine Transferrate von etwa 2,2 % ergibt, die allerdings unter der von α-Tocopherol liegt.

In den Lebern der Tiere konnte neben α- und γ-Tocopherol nur α-Tocotrienol nachgewiesen werden.

**Patentansprüche**

1. Verwendung wenigstens eines Cholesterin-Biosynthese-Hemmers zur Verringerung des Cholesteringehaltes in Geflügelei, dadurch gekennzeichnet, dass der Hemmer ein isoprenoides Strukturelement umfasst.

2. Verwendung nach Anspruch 1, dadurch gekennzeichnet, dass der Hemmer ausgewählt ist unter Tocotrienolen und Terpenoiden.

3. Verwendung nach Anspruch 2, dadurch gekennzeichnet, dass die Tocotrienole ausgewählt sind unter α-, β-, γ- und δ-Tocotrienol, den 4-Oxo-Analoga davon und den Estern dieser Verbindungen.

4. Verwendung nach Anspruch 2, dadurch gekennzeichnet, dass das Terpenoid ein pflanzliches Terpenoid ist.

5. Verwendung nach Anspruch 4, dadurch gekennzeichnet, dass das pflanzliche Terpenoid β-Ionon ist.

6. Verwendung nach Anspruch 4, dadurch gekennzeichnet, dass das pflanzliche Terpenoid ausgewählt ist unter Linalool und den Estern davon.

7. Verwendung nach Anspruch 4, dadurch gekennzeichnet, dass das pflanzliche Terpenoid ein Farnesylester ist.

8. Verwendung nach einem der vorhergehenden Ansprüche, wobei man den Hemmer in isomerenreiner Form,

optisch reiner Form, als Isomerengemisch oder Stereoisomerengemisch oder als Gemisch von Isomeren und Stereoisomeren einsetzt.

**9.** Verwendung nach einem der vorhergehenden Ansprüche, dadurch gekennzeichnet, dass man den Hemmer im Gemisch mit Futtermittel verabreicht, welches den Hemmer in einer den Cholesteringehalt von Geflügelei verringernden Konzentration enthält.

**10.** Verwendung nach einem der vorhergehenden Ansprüche zur gleichzeitigen Verringerung des Cholesteringehaltes in Geflügelei und des Triglyceridgehaltes in Geflügelei und/oder Plasma.

**11.** Futtermittelzusammensetzung für Geflügel, enthaltend in Kombination mit üblichen Futtermittelbestandteilen wenigstens einen Hemmer nach einem der Ansprüche 1 bis 8.

**12.** Futtermittelzusammensetzung nach Anspruch 11, enthaltend den jeweiligen Hemmer in einer den Cholesteringehalt von Geflügelei verringernden Konzentration.

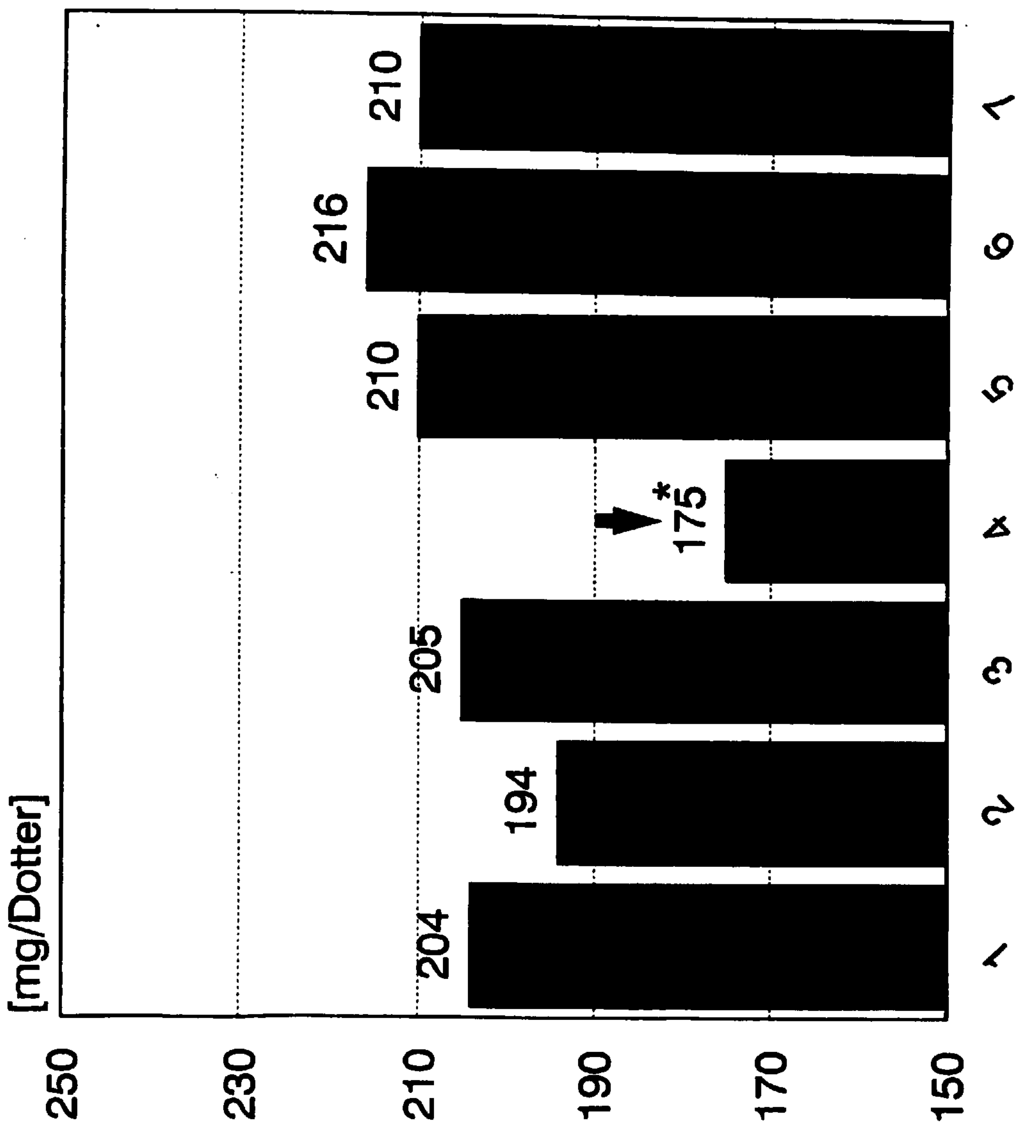
[mg/Dotter]
250
230
210
190
170
150
204
194
205
175*
210
216
210
1
2
3
4
5
6
7

Fig. 1

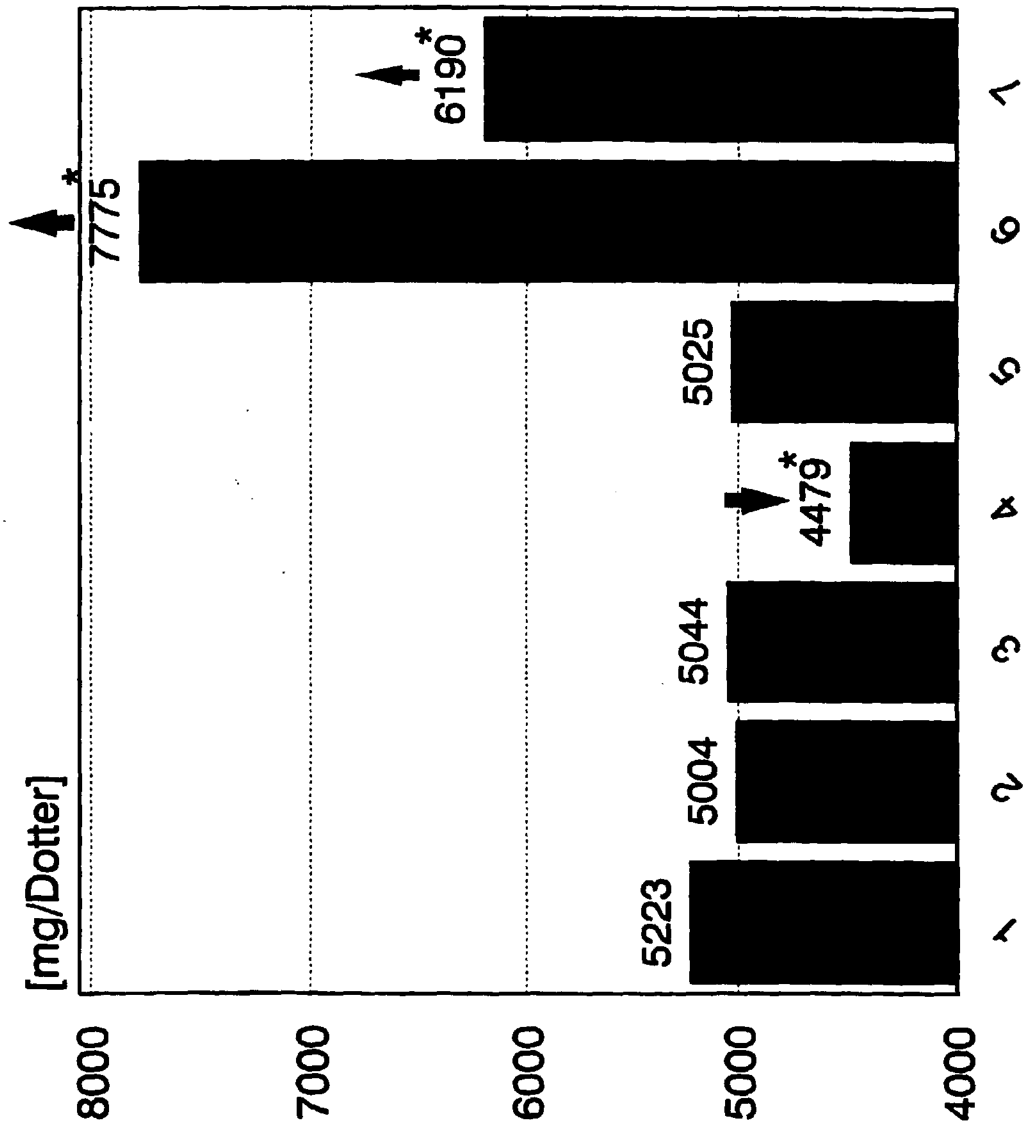
[mg/Dotter]
8000
7000
6000
5000
4000
5223
5004
5044
4479*
5025
7775*
6190*
1
2
3
4
5
6
7

Fig. 2

Europäisches Patentamt

# EUROPÄISCHER RECHERCHENBERICHT

Nummer der Anmeldung
EP 99 11 5698

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X<br><br>Y | EP 0 543 417 A (LIPOGENETICS INCORPORATED)<br>26. Mai 1993 (1993-05-26)<br>* Seite 6, Zeile 29 - Zeile 39 *<br>* Seite 7, Zeile 22 - Zeile 25 *<br>* Seite 8, Zeile 50 - Zeile 56 *<br>* Seite 10, Zeile 40 - Zeile 42 *<br>* Seite 14, Zeile 22 - Zeile 27 *<br>* Beispiele 8,12 *<br>* Ansprüche 2,4,25,26,33 *<br>--- | 1-3,8-12<br><br>1,2,4-7 | A23K1/16<br>A61K31/05 |
| Y | ELSON, C.E.: "Novel Lipids and Cancer, Isoprenoids and Other Phytochemicals"<br>DIETARY FATS, LIPIDS, HORMONES, AND TUMORGENESIS,1996, Seiten 71-86, XP002067388<br>NEW YORK US<br>* Seite 71 - Seite 72; Abbildung 1 *<br>--- | 1,2,4-7 | |
| X | HOOD, R.L. ET AL.: "The Effect of Dietary Monoterpenes on the Cholesterol Level of Eggs"<br>POULTRY SCIENCE,<br>Bd. 57, Nr. 1, 1978, Seiten 304-306, XP000856191<br>CHAMPAIGN, IL, US<br>ISSN: 0032-5791<br>* das ganze Dokument *<br>---<br>-/-- | 1,2,4,6,<br>8,10,11 | RECHERCHIERTE SACHGEBIETE (Int.Cl.7)<br>A61K<br>A23K |

Der vorliegende Recherchenbericht wurde für alle Patentansprüche erstellt

| Recherchenort | Abschlußdatum der Recherche | Prüfer |
|---|---|---|
| DEN HAAG | 22. November 1999 | Lepretre, F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

Europäisches Patentamt

# EUROPÄISCHER RECHERCHENBERICHT

Nummer der Anmeldung
EP 99 11 5698

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | YU, S.G., ET AL.: "Dietary beta-Ionone Suppresses Hepatic 3-Hydroxy-3-methylglutaryl Coenzyme A Reductase Activity" JOURNAL OF AGRICULTURAL AND FOOD CHEMISTRY., Bd. 42, Nr. 7, 1994, Seiten 1493-1496, XP002122914 AMERICAN CHEMICAL SOCIETY. WASHINGTON., US ISSN: 0021-8561 | 11 | |
| A | * Seite 1494; Tabelle 1 * * Seite 1495; Tabelle 3 * --- | 4,5 | |
| X | DATABASE BIOSIS 'Online! BIOSCIENCES INFORMATION SERVICE, PHILADELPHIA, PA, US XP002122917 | 11 | |
| A | * Zusammenfassung * & PEARCE, B.C., ET AL.: "Inhibitors of cholesterol biosynthesis." JOURNAL OF MEDICINAL CHEMISTRY., Bd. 37, Nr. 4, 1994, Seiten 526-541, AMERICAN CHEMICAL SOCIETY. WASHINGTON., US ISSN: 0022-2623 --- | 4,7 | RECHERCHIERTE SACHGEBIETE (Int.Cl.7) |
| X | US 5 296 508 A (PEARCE) 22. März 1994 (1994-03-22) | 11 | |
| A | * Spalte 2, Zeile 22 - Zeile 25 * * Spalte 2, Zeile 51 - Zeile 56 * * Spalte 4, Zeile 1 * * Tabellen 1,6 * --- | 4,7 | |
| X | US 4 603 142 A (BURGER ET AL.) 29. Juli 1986 (1986-07-29) * Spalte 4, Zeile 66 - Spalte 5, Zeile 1 * * Tabellen 1-4 * * Ansprüche 3,4 * --- -/-- | 11 | |

Der vorliegende Recherchenbericht wurde für alle Patentansprüche erstellt

| Recherchenort | Abschlußdatum der Recherche | Prüfer |
|---|---|---|
| DEN HAAG | 22. November 1999 | Lepretre, F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 99 11 5698

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| --- | --- | --- | --- |
| D,X | US 5 217 992 A (WRIGHT ET AL.)<br>8. Juni 1993 (1993-06-08)<br>* Beispiel 7; Tabelle 5 *<br>* Ansprüche 1-6 *<br>--- | 11 | |
| X | DATABASE FSTA 'Online!<br>INTERNATIONAL FOOD INFORMATION SERVICE (IFIS), FRANFURT/MAIN, DE<br>XP002122918<br>* Zusammenfassung *<br>& QURESHI, A.A. ET AL.: "Effects of brewers grain and other cereals on lipid metabolism in chickens."<br>NUTRITION RESEARCH.,<br>Bd. 11, Nr. 2/3, 1991, Seiten 159-168, XX, XX<br>ISSN: 0271-5317<br>--- | 11 | |
| A | DATABASE FSTA 'Online!<br>INTERNATIONAL FOOD INFORMATION SERVICE (IFIS), FRANFURT/MAIN, DE<br>XP002122919<br>* Zusammenfassung *<br>& SUGIYAMA, K. ET AL.: "Isolation of plasma cholesterol-lowering components from ningyotake (Polyporus confluens) mushroom."<br>JOURNAL OF NUTRITIONAL SCIENCE AND VITAMINOLOGY.,<br>Bd. 38, Nr. 4, 1992, Seiten 335-342,<br>ISSN: 0301-4800<br>----- | 7 | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
| --- | --- | --- |
| DEN HAAG | 22. November 1999 | Lepretre, F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 99 11 5698

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-11-1999

| Im Recherchenbericht angeführtes Patentdokument | Datum der Veröffentlichung | Mitglied(er) der Patentfamilie | Datum der Veröffentlichung |
|---|---|---|---|
| EP 543417 A | 26-05-1993 | AU 670557 B | 25-07-1996 |
| | | AU 3150293 A | 15-06-1993 |
| | | CA 2124086 A | 27-05-1993 |
| | | CN 1073357 A | 23-06-1993 |
| | | JP 7504887 T | 01-06-1995 |
| | | MX 9206735 A | 01-05-1993 |
| | | NO 941929 A | 13-07-1994 |
| | | SG 48108 A | 17-04-1998 |
| | | WO 9309777 A | 27-05-1993 |
| | | US 5591772 A | 07-01-1997 |
| | | US 5908940 A | 01-06-1999 |
| | | US 5821264 A | 13-10-1998 |
| | | US 5919818 A | 06-07-1999 |
| US 5296508 A | 22-03-1994 | US 5204373 A | 20-04-1993 |
| US 4603142 A | 29-07-1986 | KEINE | |
| US 5217992 A | 08-06-1993 | CA 2026713 A | 05-04-1991 |
| | | JP 3246222 A | 01-11-1991 |
| | | US 5348974 A | 20-09-1994 |
| | | EP 0421419 A | 10-04-1991 |

EPO FORM P0461

Für nähere Einzelheiten zu diesem Anhang : siehe Amtsblatt des Europäischen Patentamts, Nr.12/82